# EUROPEAN PATENT APPLICATION

(11) **EP 4 382 162 A1**
(43) Date of publication of application: **12.06.2024**
(21) Application number: 22852352.8
(22) Date of filing: 05.08.2022
(51) Int. Cl.: A61N 1/365, A61N 1/362, A61N 1/36

(54) **PULSE STIMULATION DEVICE AND METHOD, AND MEDICAL APPARATUS**

(30) Priority: 05.08.2021 CN 202110897971
(71) Applicant: United Innomed (Shanghai) Limited, Shanghai 201315 (CN)
(72) Inventor: WANG, Li, Shanghai 201315 (CN)
(74) Representative: dompatent von Kreisler Selting Werner - Partnerschaft von Patent- und Rechtsanwälten mbB
(86) International application number: PCT/CN2022/110614
(87) International publication number: WO 2023/011641

(57) **Abstract**

A pulse stimulation device and a medical apparatus. The device comprises an R-wave sensing module (1), a cardiac stimulation pulse generator (2) and at least one control electrode (3); the R-wave sensing module (1) is used for acquiring a surface electrocardiogram and/or a myocardial electrocardiogram, and for acquiring an R-wave sensing time of the appearance of an R wave, so as to determine a pulse delivery time for each preset stimulation position, and the pulse delivery time corresponds to the duration from the moment when the R wave is detected to the moment when the delivery of a cardiac stimulation pulse is triggered; and the cardiac stimulation pulse generator (2) is used for delivering the cardiac stimulation pulse to the control electrode (3) according to the pulse delivery time. In this way, multi-part electrical stimulation of the left ventricle and the right ventricle is realized.

## Description

The present application claims the right of the priority of Chinese patent application 2021108979710 filed on August 5, 2021. The contents of the above Chinese patent application are incorporated herein by reference in its entirety.

### TECHNICAL FIELD

The present disclosure relates to the technical field of medical equipment, in particular to a pulse stimulation device and method, and a medical apparatus.

### BACKGROUND

The CCM (cardiac contractility modulation) device available on the market is a standalone, implantable, structurally complex, and expensive apparatus basically used for patients with chronic heart failure. It involves the implantation of two bipolar leads in the interventricular septum of the right ventricle for sensing the local myocardial potential and delivering the cardiac stimulation pulse within a certain period of time after sensing (within the absolute refractory period), so as to increase myocardial contractility. At this point, the myocardial stimulation is not acting directly on the left ventricle, which is the ventricle most in need of increased contractility. Although the CCM applied to the interventricular septum of the right ventricle has an overall effect on cardiac contractility and cardiac function (including left ventricular contractility), studies have shown that such overall effect is not direct but rather driven by the impact on the local myocardium through local stimulation for the interventricular septum of the right ventricle.

Currently, CCM stimulation is only used in implantable devices for patients with chronic heart failure, whose long-term treatment is achieved through single-site stimulation of the interventricular septum of the right ventricle. However, when a patient's cardiac function deteriorates rapidly and blood pressure drops (e.g., during acute heart failure episodes), the patient does not need or benefit from long-term use of an implantable device, or only needs relatively short-term support for cardiac function until the underlying cause is eliminated and/or cardiac function is restored. Therefore, the existing implantable CCM and its single-site stimulation cannot meet the needs for acute and/or short-term support (in terms of duration and degree of cardiac/circulatory support), and the provision of acute and short-term (days) cardiac/circulatory support is crucial for the survival of patients (e.g., during acute heart failure episodes) or those being resuscitated in emergency situations such as the emergency room or ambulance, where any additional (non-pharmacological) cardiac circulatory support can mean the difference between "life and death" for the patient.

### CONTENT OF THE PRESENT INVENTION

The technical problem to be solved by the present disclosure is to provide a pulse stimulation device and method, and a medical apparatus in order to overcome the drawbacks in the prior art that the existing implantable CCM stimulation mode is only suitable for a patient with chronic heart failure and cannot meet the acute and/or short-term need of a patient, and single-site electrical stimulation may not be adequate for the circulatory support needed by the heart of a patient.

The present disclosure solves the above technical problem by the following technical solutions:
The present disclosure provides a pulse stimulation device, comprising an R-wave sensing module, a cardiac stimulation pulse generator, and at least one control electrode, wherein each control electrode corresponds to a different preset stimulation position;
the control electrode is electrically connected to the R-wave sensing module and the cardiac stimulation pulse generator, respectively, and the R-wave sensing module is communicatively connected to the cardiac stimulation pulse generator;
the R-wave sensing module is used to obtain a surface electrocardiogram and/or obtain a myocardial electrocardiogram by collecting an electrocardiogram signal based on the control electrode, obtain an R-wave sensing time of a corresponding R-wave occurrence based on the surface electrocardiogram and/or the myocardial electrocardiogram, and determine a pulse delivery time corresponding to each preset stimulation position according to the R-wave sensing time;
the cardiac stimulation pulse generator is used to deliver a cardiac stimulation pulse to each corresponding control electrode according to each pulse delivery time.

In this solution, by obtaining the R-wave sensing time of the corresponding R-wave occurrence based on the surface electrocardiogram and/or the myocardial electrocardiogram to determine the pulse delivery time corresponding to the preset stimulation position, it achieves the acute and/or short-term need of a patient, and solves the problem of inadequate circulatory support provided by single-site electrical stimulation to the heart of a patient, and ensures the timeliness and reliability of cardiac stimulation pulse triggering, thereby effectively ensuring the safety of the patient.

Optionally, the device comprises at least two control electrodes, and different control electrodes are used for being implanted at different preset stimulation positions in the left ventricular myocardium and/or right ventricular myocardium of the patient.

In this solution, by setting at least two control electrodes to realize stimulation of multiple different positions in the myocardium of the left and right ventricles, it can more effectively improve the overall cardiac contractility (especially the left ventricular contractility) of the patient, compared with the existing mode of merely stimulating the interventricular septum of the right ventricle. For patients with acute heart failure and/or short-term significant decline in ventricular function, multi-site stimulation can better enhance cardiac contractility and improve cardiac ejection function.

Optionally, the device further comprises a pulse control module, and the pulse control module is communicatively connected to the cardiac stimulation pulse generator;
the pulse control module is used to generate a pulse delivery pattern and send the pulse delivery pattern to the cardiac stimulation pulse generator;
the cardiac stimulation pulse generator is used to deliver a cardiac stimulation pulse to each corresponding control electrode based on the pulse delivery pattern and each pulse delivery time upon the occurrence of an R wave in the surface electrocardiogram and/or the myocardial electrocardiogram.

In this solution, by presetting different pulse delivery patterns in the pulse control module, in an actual pulse delivery scenario, the pulse delivery pattern can be determined based on manual selection by an operator, presetting of a fixed pulse pattern, or random selection, so as to automatically realize the pulse delivery for different preset stimulation positions. It is certain that the pulse delivery pattern can also be dynamically adjusted according to actual needs.

Optionally, the pulse delivery pattern comprises delivering a cardiac stimulation pulse to the control electrode corresponding to each preset stimulation position in a synchronized manner and in a set or random order, based on the R wave in the surface electrocardiogram and/or the myocardial electrocardiogram.

In this solution, the pulse can be delivered in a synchronized manner and in a set or random order, so as to satisfy various pulse delivery needs as much as possible for more pulse stimulation scenarios, which ensures the effectiveness of pulse stimulation for the patient while improving the experience of the patient.

Optionally, the pulse control module is also used to generate a stimulation combination corresponding to different preset stimulation positions based on a set number of the preset stimulation positions, using a set construction rule or a random combination;
wherein the stimulation combination comprises at least two stimulation units, and at least one of the stimulation units corresponds to two or more of the preset stimulation positions where the pulse is delivered in a synchronized manner;
the pulse delivery pattern comprises delivering a cardiac stimulation pulse to the control electrode corresponding to the preset stimulation position in accordance with a set or random order and the stimulation combination, based on the R wave in the surface electrocardiogram and/or the myocardial electrocardiogram.

In this solution, each preset stimulation position is no longer considered separately, but different stimulation combinations are formed based on multiple preset stimulation positions. The pulse is delivered in a synchronized manner for different preset stimulation positions corresponding to the same stimulation combination, and different stimulation combinations use a set or random order of pulse delivery for pulse stimulation, so as to satisfy the needs of more pulse stimulation scenarios, further ensuring the safety of the patient.

Optionally, the cardiac stimulation pulse is successively delivered to the corresponding preset stimulation position in a set or random order to achieve a preset number of heartbeats.

In this solution, control electrodes are placed at multiple sites in the left ventricle and/or right ventricle to provide simultaneous or sequential cardiac electrical stimulation (CCM) of multiple sites for electrical circulatory support (ECS). It can more effectively improve the overall cardiac contractility (especially the left ventricular contractility) of the patient, compared with the mode of merely stimulating the interventricular septum of the right ventricle in the prior art. For patients with acute heart failure and/or short-term significant decline in ventricular function, multi-site stimulation can better enhance cardiac contractility and improve cardiac ejection function.

Optionally, the preset stimulation position comprises at least one of an inner wall of the interventricular septum of the left ventricle and/or right ventricle, an interventricular sulcus of the outer wall of the ventricle, an lateral wall of the left ventricle, an anterolateral wall of the left ventricle, a posterolateral wall of the left ventricle, an inner wall of a right ventricular free wall, an outer wall of the right ventricular free wall, a right ventricular apex, and a left ventricular apex.

In this solution, the control electrodes are separately set in the above-mentioned positions of the left ventricle and/or right ventricle, or the control electrodes are set in some of the positions according to actual stimulation needs to ensure the reliability of cardiac pulse stimulation as much as possible.

Optionally, the R-wave sensing module is used to obtain a surface electrocardiogram and a myocardial electrocardiogram based on the control electrode, and to obtain a first R-wave sensing time of the R-wave occurrence based on the myocardial electrocardiogram and a second R-wave sensing time of the R-wave occurrence based on the surface electrocardiogram;
the R-wave sensing module is also used to calculate a first pulse delivery time corresponding to each preset stimulation position based on the first R-wave sensing time;
the R-wave sensing module is also used to calculate a second pulse delivery time corresponding to each preset stimulation position based on the first R-wave sensing time and the second R-wave sensing time.

The cardiac stimulation pulse generator is used to deliver a cardiac stimulation pulse to each corresponding control electrode based on the first pulse delivery time and/or the second pulse delivery time.

In this solution, the first R-wave sensing time of the R-wave occurrence in the myocardial electrocardiogram is obtained, and the first pulse delivery time corresponding to the preset stimulation position is calculated; the second R-wave sensing time of the R-wave occurrence in the surface electrocardiogram is obtained, then the second pulse delivery time corresponding to the preset stimulation position is calculated based on the first R-wave sensing time and the second R-wave sensing time, and then the pulse is delivered based on the first pulse delivery time and/or the second pulse delivery time, that is, a solution is proposed to determine the pulse delivery time based on the R wave in the myocardial electrocardiogram and the R wave in the surface electrocardiogram, which effectively ensures the timeliness and reliability of pulse stimulation.

Optionally, the R-wave sensing module is also used to calculate the first pulse delivery time corresponding to each preset stimulation position based on a preset duration, with the first R-wave sensing time as a reference zero point;

the R-wave sensing module is also used to calculate a first time difference between the second R-wave sensing time and the first R-wave sensing time of each preset stimulation position, and to calculate the second pulse delivery time corresponding to each preset stimulation position based on the first time difference and the preset duration, with the second R-wave sensing time as a reference zero point.

Optionally, the cardiac stimulation pulse generator is used to maintain the first time difference after obtaining the second pulse delivery time, so as to maintain delivering a cardiac stimulation pulse to the corresponding control electrode based on the second pulse delivery time.

Optionally, the device further comprises a time update module;
the time update module is used to periodically or irregularly update the first time difference, so as to update the second pulse delivery time based on the updated first time difference;
the cardiac stimulation pulse generator is used to deliver a cardiac stimulation pulse to the corresponding control electrode based on the updated second pulse delivery time.

In this solution, the second pulse delivery time (at which time the myocardial electrical stimulation can be continued or terminated) can be updated periodically or irregularly according to actual needs, and then the myocardial electrical stimulation can be continued based on an updated trigger time, so as to achieve a more flexible electrical stimulation effect and satisfy the needs of more pulse electrical stimulation scenarios.

Optionally, the R-wave sensing module is also used to obtain the myocardial electrocardiograms corresponding to all other remaining preset stimulation positions when pacing is performed at one preset stimulation position, and to obtain a new first R-wave sensing time of the R-wave occurrence in the myocardial electrocardiogram and a new second R-wave sensing time of the R-wave occurrence in the surface electrocardiogram;
the R-wave sensing module is also used to calculate a new first pulse delivery time corresponding to each preset stimulation position based on the new first R-wave sensing time and the preset duration;
the R-wave sensing module is also used to calculate a new first time difference between the new second R-wave sensing time and the new first R-wave sensing time of each preset stimulation position, and to calculate a new second pulse delivery time corresponding to each preset stimulation position based on the new first time difference and the preset duration, with the new second R-wave sensing time as a reference zero point.

In this solution, when pacing is performed via one control electrode, it is necessary to recalculate a new R-wave sensing time of the R wave in the myocardial electrocardiogram as well as the R wave in the surface electrocardiogram in this pulse stimulation scenario, and then to calculate the corresponding first pulse delivery time and the second pulse delivery time, respectively, so as to realize timely delivery of pulse stimulation in this scenario.

Optionally, the R-wave sensing module is used to obtain multiple first R-wave sensing times corresponding to the R-wave occurrence in the myocardial electrocardiogram at multiple preset stimulation positions, to select one first R-wave sensing time as a reference zero point, to calculate a second difference between each first R-wave sensing time following the reference zero point and the reference zero point, and to calculate the first pulse delivery time corresponding to each preset stimulation position based on the second difference and the preset duration.

In this solution, the pulse delivery time of each pulse stimulation position can be obtained by arbitrarily selecting one first R-wave sensing time (LS) as a reference zero point, using the reference zero point as the basis for the delivery time of other later stimulation positions, calculating the difference between the delivery time and the reference zero point, and adding the preset duration, so as to achieve the timely and effective pulse delivery based only on the myocardial electrocardiogram without relying on the surface electrocardiogram, making the process of controlling the pulse stimulation more flexible and adaptable to more usage scenarios.

Optionally, the R-wave sensing module is used to randomly select one first R-wave sensing time from multiple first R-wave sensing times with times of occurrence, or to select the first R-wave sensing time with the earliest time of occurrence as the reference zero point.

Optionally, the first R-wave sensing time of the R-wave occurrence obtained in the myocardial electrocardiogram and the second R-wave sensing time of the R-wave occurrence obtained in the surface electrocardiogram correspond to the same heartbeat.

In this solution, in order to ensure the effectiveness of pulse stimulation, all R waves (i.e., R waves in the myocardial electrocardiogram and R waves in the surface electrocardiogram) are sensed at the same heartbeat; that is, the time difference between the first R-wave sensing time of the R-wave occurrence obtained in the myocardial electrocardiogram and the second R-wave sensing time of the R-wave occurrence obtained in the surface electrocardiogram must be less than a certain value, so that there is practical significance of pulse stimulation, otherwise the reliability of pulse stimulation cannot be ensured.

Optionally, the control electrode is electrically connected to the R-wave sensing module and the cardiac stimulation pulse generator using a unipolar lead or a bipolar lead.

The present disclosure also provides a pulse stimulation method, which is implemented using the pulse stimulation device as described above, comprising:
obtaining a surface electrocardiogram and/or obtaining a myocardial electrocardiogram by collecting an electrocardiogram signal based on the control electrode;
obtaining an R-wave sensing time of an R-wave occurrence based on the surface electrocardiogram and/or the myocardial electrocardiogram;
determining a pulse delivery time corresponding to each preset stimulation position based on the R-wave sensing time; and
delivering a cardiac stimulation pulse to each corresponding control electrode based on each pulse delivery time.

The present disclosure also provides a medical apparatus, comprising the pulse stimulation device as described above.

On the basis of not violating common knowledge in the art, the preferred conditions can be combined arbitrarily to obtain preferred examples of the present disclosure.

The positive progressive effect of the present disclosure is that:
(1) Placing control electrodes (e.g., corresponding to stimulation and/or pacing functions) at multiple sites in the left ventricle and/or right ventricle enables simultaneous or sequential cardiac electrical stimulation (CCM) to multiple sites to provide a circulatory support to the heart; it can more effectively improve the overall cardiac contractility (especially the left ventricular contractility) of the patient, compared with the existing mode of merely stimulating the interventricular septum of the right ventricle. For patients with acute heart failure and/or short-term significant decline in ventricular function, multi-site stimulation can better enhance cardiac contractility and improve cardiac ejection function. For example, in patients who have just undergone cardiac surgery, leads are implanted at different positions in their left ventricular myocardium and/or right ventricular myocardium (e.g., anterior/posterior position of the left ventricle on the epicardial surface, interventricular sulcus, right ventricular free wall). In other patients, leads can be placed transvenously within the right ventricle (e.g., in the interventricular septum, apex, or free wall) and/or leads can be placed on the endocardium of the left ventricle (e.g., in the interventricular septum, apex, or free wall) via the interatrial septum or arteries or the epicardial surface of the left ventricle (via electrodes placed in the coronary veins or arteries), among others.
(2) Cardiac electrical stimulation (CCM) mode (mechanism, timing, etc.): a. The stimulation mechanism is a mechanism for multi-site cardiac electrical stimulation (in a synchronized or sequential manner), e.g., stimulation triggered at all electrodes in one cardiac cycle (synchronized stimulation), or stimulation triggered at each electrode in multiple cardiac cycles in a set or random order (sequential stimulation); b. the triggering mechanism uses the time of R-wave sensing representing localized ventricular myoelectric activity and/or R-wave sensing representing overall ventricular myoelectric activity as the trigger point. In the latter case, the temporal relationship between the overall and localized ventricular myoelectric R waves becomes part of the trigger time. The stimulation mode (mechanism, timing, etc.) can be dynamically adjusted according to the actual situation to adapt to the changing heart rate and overall cardiac condition of the patient, i.e., it can be adaptively adjusted according to the dynamic change of each patient's own cardiac condition, so as to effectively improve cardiac function.
(3) CCM transmission can be achieved with a simple unipolar lead, instead of having to use two unipolar or bipolar leads at one cardiac position, thereby simplifying the system architecture and reducing the cost.

### BRIEF DESCRIPTION OF THE DRAWINGS

Figure 1 shows a schematic structural diagram of a pulse stimulation device according to Example 1 of the present disclosure.
Figure 2 shows a schematic diagram of R-wave triggering corresponding to electrocardiogram (ECG) and electrograms (EGMs) during CCM transmission according to Example 1 of the present disclosure.
Figure 3 shows a schematic structural diagram of a pulse stimulation device according to Example 2 of the present disclosure.
Figure 4 shows a schematic diagram of R-wave triggering corresponding to ECG and EGMs during CCM sequential transmission according to Example 2 of the present disclosure.
Figure 5 shows a flow chart of a pulse stimulation method according to Example 3 of the present disclosure.

### DETAILED DESCRIPTION OF THE PREFERRED EMBODIMENT

The present disclosure is further illustrated below by means of examples, but the present disclosure is not limited to the scope of the examples.

### Example 1

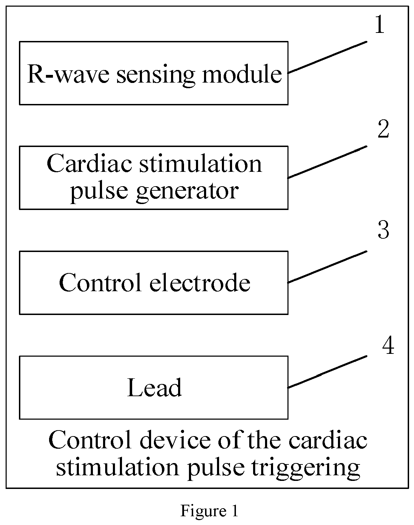

As shown in Figure 1, a pulse stimulation device of this example comprises an R-wave sensing module 1, a cardiac stimulation pulse generator 2, and at least one control electrode 3, wherein the control electrode 3 is electrically connected to the R-wave sensing module 1 and the cardiac stimulation pulse generator 2, respectively, via a lead 4, and the R-wave sensing module 1 is communicatively connected to the cardiac stimulation pulse generator 2.

Preferably, the device comprises at least two control electrodes, and different control electrodes are used for being implanted at different preset stimulation positions in the left ventricular myocardium and/or right ventricular myocardium of a patient.

By setting at least two control electrodes to realize stimulation of multiple different positions in the left ventricular myocardium and/or right ventricular myocardium, it can more effectively improve the overall cardiac contractility (especially the left ventricular contractility) of the patient, compared with the existing mode of merely stimulating the interventricular septum of the right ventricle. For patients with acute heart failure and/or short-term significant decline in ventricular function, multi-site stimulation can better enhance cardiac contractility and improve cardiac ejection function. It is certain that the number of control electrodes can be reset and adjusted according to actual scenario needs.

Specifically, different preset stimulation positions include, but are not limited to, an inner wall of the interventricular septum of the left ventricle and/or right ventricle, an interventricular sulcus of the outer wall of the ventricle, an lateral wall of the left ventricle, an anterolateral wall of the left ventricle, a posterolateral wall of the left ventricle, an inner wall of a right ventricular free wall, an outer wall of the right ventricular free wall, a right ventricular apex, and a left ventricular apex.

The control electrodes are separately set in the above-mentioned positions of the left ventricle and/or right ventricle, or the control electrodes are set in some of the positions according to actual stimulation needs to ensure the reliability of cardiac pulse stimulation as much as possible.

The R-wave sensing module 1 is used to obtain a surface electrocardiogram (ECG) and/or obtain a myocardial electrocardiogram (EGM) by collecting an electrocardiogram signal based on the control electrode 3. Since the control electrode can be located in either the ventricular endocardium or the ventricular epicardium, the myocardial electrocardiogram (EGM) encompasses signals originating from either the ventricular cavity or the outer wall of the ventricle.

Specifically, the R-wave sensing module 1 is used to obtain an R-wave sensing time of an R-wave occurrence based on the surface electrocardiogram and/or the myocardial electrocardiogram, and determine a pulse delivery time of cardiac stimulation (or CCM electrical stimulation) corresponding to each preset stimulation position based on the R-wave sensing time. The pulse delivery time corresponds to the duration from sensing the R wave to triggering cardiac stimulation pulse delivery, and CCM transmission at various positions or sites is triggered by R-wave sensing of the local myocardial electrocardiogram (EGM) or R-wave sensing of the global surface electrocardiogram (ECG).

The cardiac stimulation pulse generator 2 is used to deliver a cardiac stimulation pulse to each corresponding control electrode 3 according to each pulse delivery time.

In an implementable embodiment, the R-wave sensing module is used to obtain a surface electrocardiogram and/or obtain a myocardial electrocardiogram based on the control electrode, and to obtain a first R-wave sensing time of the R-wave occurrence based on the myocardial electrocardiogram and a second R-wave sensing time of the R-wave occurrence based on the surface electrocardiogram;
It should be noted that in this example, the first R-wave sensing time of the R-wave occurrence obtained in the myocardial electrocardiogram and the second R-wave sensing time of the R-wave occurrence obtained in the surface electrocardiogram correspond to the same heartbeat.

In order to ensure the effectiveness of pulse stimulation, all R waves (i.e., R waves in the myocardial electrocardiogram and R waves in the surface electrocardiogram) are sensed at the same heartbeat; that is, the time difference between the first R-wave sensing time of the R-wave occurrence obtained in the myocardial electrocardiogram and the second R-wave sensing time of the R-wave occurrence obtained in the surface electrocardiogram must be less than a certain value, so that there is practical significance of pulse stimulation, otherwise the reliability of pulse stimulation cannot be ensured. The R-wave sensing module is also used to calculate a first pulse delivery time corresponding to each preset stimulation position based on the first R-wave sensing time; wherein once the first R-wave sensing time is determined, the corresponding first pulse delivery time (i.e., a preset duration) is also determined and will not change.

The R-wave sensing module is also used to calculate a second pulse delivery time corresponding to each preset stimulation position based on the first R-wave sensing time and the second R-wave sensing time.

The cardiac stimulation pulse generator is used to deliver a cardiac stimulation pulse to the control electrode based on the first pulse delivery time and/or the second pulse delivery time.

During the pulse stimulation process in this example, (1) the cardiac stimulation pulse can be delivered mainly based on the R wave in the surface electrocardiogram, that is, for stimulation delivery time of each electrode, the R-wave sensing time of the surface electrocardiogram is used as the zero point of the trigger time; (2) the cardiac stimulation pulse can be delivered mainly based on the R wave in the myocardial electrocardiogram, that is, for stimulation delivery time of each electrode, the R-wave sensing time of the myocardial electrocardiogram is used as the zero point of the trigger time; (3) the cardiac stimulation pulse can be delivered based on the R wave in the surface electrocardiogram and the R wave in the myocardial electrocardiogram at the same time; the specific trigger mechanism among these three can be selected or adjusted in real time according to the actual needs of electrical stimulation.

Additionally, in order to further improve the control effect of cardiac stimulation pulse triggering, when the body surface ECG sensing is poor, it can be directly switched to EGM-based triggering; or, when the EGM sensing at one position is poor, it can be directly switched to ECG-based triggering; that is, it avoids the inability of continuous pulse stimulation due to special conditions such as incorrect sensing or poor sensing, and ensures that the therapy can be carried out successively in a timely and effective manner.

In an implementable embodiment, the R-wave sensing module 1 is used to calculate a first pulse delivery time corresponding to each preset stimulation position based on the first R-wave sensing time and the preset duration.

For the myocardial electrocardiogram (EGM) collected by the electrode 3, the first pulse delivery time is a preset duration (LPD) using the R-wave sensing time of the myocardial electrocardiogram (EGM) as a reference zero point (or trigger point), wherein the preset duration is usually 40 ms by default, and the value of the duration can be adjusted according to actual needs. The LPD (i.e., the first pulse delivery time) of each site or position is used to trigger CCM delivery at the site or position relative to the R-wave sensing time of the myocardial electrocardiogram.

the R-wave sensing module 1 is also used to calculate a first time difference between the second R-wave sensing time and the first R-wave sensing time, and to calculate a second pulse delivery time corresponding to each preset stimulation position using the second R-wave sensing time as a reference zero point (or trigger point) based on the first time difference and the preset duration.

The first R-wave sensing time of the R-wave occurrence in the myocardial electrocardiogram is obtained, and the first pulse delivery time corresponding to the preset stimulation position is calculated; the second R-wave sensing time of the R-wave occurrence in the surface electrocardiogram is obtained, then the second pulse delivery time corresponding to the preset stimulation position is calculated based on the first R-wave sensing time and the second R-wave sensing time, and the pulse is delivered based on the first pulse delivery time and/or the second pulse delivery time, that is, a solution is proposed to determine the pulse delivery time based on the R wave in the myocardial electrocardiogram and the R wave in the surface electrocardiogram, which effectively ensures the timeliness and reliability of pulse stimulation.

In other words, for CCM triggered by the R wave in EGM, the duration from R wave to delivery for each stimulation position is fixed (e.g., a preset duration of 40 ms). For CCM triggered by the R wave in ECG, the duration for each stimulation position is variable and different (i.e., determined by a fixed preset duration of 40 ms and a time difference based on position change).

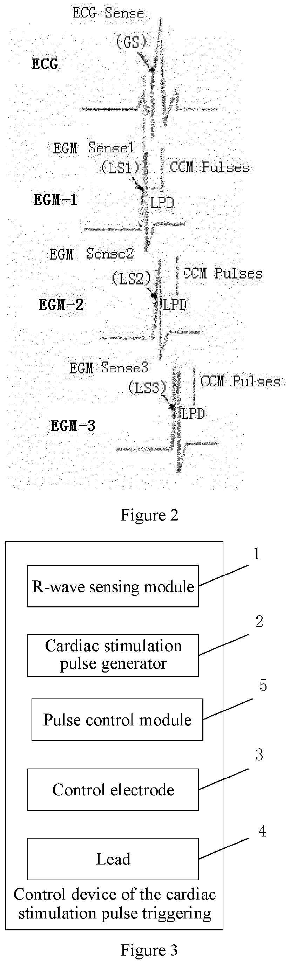

As shown in Figure 2, when the patient is in sinus rhythm, for the surface electrocardiogram (ECG), the time difference (GLSD) between the corresponding R-wave sensing time of the surface electrocardiogram (ECG) and the corresponding R-wave sensing time of the local myocardial electrocardiogram (EGM) is first calculated, which represents the time-sensitive value of the myocardial electrical activity corresponding to the myocardial site/position relative to the entire cardiac electrical activity; the R-wave sensing time of the surface electrocardiogram (ECG) is used to trigger a pulse delivery time (GPD), which will be an addition of the corresponding time difference (GLSD) and the pulse delivery time of the myocardial electrocardiogram (LPD), that is, GPD = GLSD + LPD. Additionally, the GLSD and GPD corresponding to each site can be measured and averaged over several self heartbeats (5 consecutive self heartbeats by default, ranging from 3 to 12). The GPD (i.e., the second pulse delivery time) of each site or position is used to trigger CCM delivery at the site or position relative to the R-wave sensing time of the surface electrocardiogram. In this example, the step is called a set-up period.

In an implementable embodiment, the cardiac stimulation pulse generator is used to maintain the first time difference after obtaining the second pulse delivery time for each position, so as to maintain delivering a cardiac stimulation pulse to the corresponding control electrode based on the second pulse delivery time.

That is, in this example, after calculating the second pulse delivery time, there is no need to calculate it again before each electrical stimulation output, and the electrical stimulation output time can be directly triggered by the surface electrocardiogram without the need to sense the R wave of the myocardial electrocardiogram, so as to achieve the effect of cardiac electrical stimulation while effectively shortening the data processing time and improving the control efficiency of cardiac stimulation pulse triggering.

In an implementable embodiment, the device of this example further comprises a time update module.

The time update module is used to periodically or irregularly update the first time difference, so as to update the second pulse delivery time based on the updated first time difference;
the cardiac stimulation pulse generator is used to deliver a cardiac stimulation pulse to the corresponding control electrode based on the updated second pulse delivery time.

That is, in this example, the second pulse delivery time (at which time the myocardial electrical stimulation can be continued or terminated) can be updated periodically or irregularly according to actual needs, and then the myocardial electrical stimulation can be continued based on an updated trigger time, so as to achieve a more flexible electrical stimulation effect and satisfy the needs of more pulse electrical stimulation scenarios.

In this example, the effect of enhancing or maximizing the circulatory support to the heart is achieved by setting the stimulation position and stimulation mechanism.

It is certain that the control scheme for CCM stimulation in this example needs to be automatically suspended in certain special cases, such as when the patient's heart rate is detected to be too fast (e.g., higher than 120 beats per minute) or the patient has a PVC (premature ventricular contraction), so as to ensure the safety of the patient's cardiac stimulation support.

In this example, placing control electrodes (e.g., corresponding to stimulation and/or pacing functions) at multiple sites in the left ventricle and/or right ventricle enables cardiac electrical stimulation (CCM) of multiple sites; it can more effectively improve the overall cardiac contractility (especially the left ventricular contractility) of the patient, compared with the existing mode of merely stimulating the interventricular septum of the right ventricle. For patients with acute heart failure and/or short-term significant decline in ventricular function, multi-site stimulation can better enhance cardiac contractility and improve cardiac ejection function.

### Example 2

As shown in Figure 3, a pulse stimulation device of this example is a further improvement of example 1, specifically:
The pulse stimulation device of this example further comprises a pulse control module 5, which is communicatively connected to a cardiac stimulation pulse generator 2.

The pulse control module 5 is used to generate a pulse delivery pattern and send the pulse delivery pattern to the cardiac stimulation pulse generator 2.

The cardiac stimulation pulse generator 2 is used to deliver a cardiac stimulation pulse to each corresponding control electrode 3 based on the pulse delivery pattern and a pulse delivery time upon the occurrence of an R wave in a surface electrocardiogram or a myocardial el ectrocardi ogram.

By presetting different pulse delivery patterns in the pulse control module, in an actual pulse delivery scenario, the pulse delivery pattern can be determined based on manual selection by an operator, presetting of a fixed pulse pattern, or random selection, so as to automatically realize the pulse delivery for different preset stimulation positions. It is certain that the pulse delivery pattern can also be dynamically adjusted according to actual needs.

In an implementable embodiment, the pulse delivery pattern comprises delivering a cardiac stimulation pulse to the control electrode corresponding to each preset stimulation position in a synchronized manner and in a set or random order, based on the R wave in the surface electrocardiogram and/or the myocardial electrocardiogram.

The cardiac stimulation pulse is successively delivered to the corresponding preset stimulation position in a set or random order to achieve a preset number of heartbeats.

The pulse delivery pattern is triggered by the global R wave of the surface electrocardiogram or the local R wave of each site with an delivery mechanism (i.e., delivering a cardiac stimulation pulse to each preset stimulation position in a synchronized manner and in a set or random order) or a set-up period (i.e., measurement and calculation of GLSD corresponding to each electrode position).

The pulse can be delivered in a synchronized manner and in a set or random order, so as to satisfy various pulse delivery needs as much as possible for more pulse stimulation scenarios, which ensures the effectiveness of pulse stimulation for a patient while improving the experience of the patient.

CCM stimulation of a position or site can be triggered and transmitted "simultaneously" (i.e., in the same cardiac cycle) according to the corresponding time of each site after the same R wave (called synchronization), or it can be sequentially transmitted at multiple sites on multiple R waves; when transmitted in a set order, CCM stimulation is triggered and transmitted at one site according to the corresponding time of the site after the R wave, and then transmitted at the next site after the next R wave, and so on until all sites are covered by the transmission. After the same R wave, CCM stimulation may occur at one or more than one site according to the corresponding time of each site (but not all sites, otherwise it will be synchronized). Additionally, the site-specific sequence of receiving CCM stimulation can be specifically designed (which can be programmed by a staff member with relevant authority, such as a physician) or randomized. During sequential transmission, each preset site can be stimulated once or more than once (e.g., six times, i.e., six cardiac cycles) before moving on to the next preset site to be stimulated, and so on.

In an implementable embodiment, the pulse control module is also used to generate a stimulation combination corresponding to different preset stimulation positions based on a set number of the preset stimulation positions, using a set construction rule or a random combination;
wherein the stimulation combination comprises at least two stimulation units, and at least one of the stimulation units corresponds to two or more of the preset stimulation positions where the pulse is delivered in a synchronized manner;
the pulse delivery pattern comprises delivering a cardiac stimulation pulse to the control electrode corresponding to the preset stimulation position in accordance with a set or random order and the stimulation combination, based on the R wave in the surface electrocardiogram and/or the myocardial electrocardiogram.

The cardiac stimulation pulse is successively delivered to the corresponding preset stimulation position in a set or random order to achieve a preset number of heartbeats.

Each preset stimulation position is no longer considered separately, but different stimulation combinations are formed based on multiple preset stimulation positions. The pulse is delivered in a synchronized manner for different preset stimulation positions corresponding to the same stimulation combination, and different stimulation combinations use a set or random order of pulse delivery for pulse stimulation, so as to satisfy the needs of more pulse stimulation scenarios, further ensuring the safety of the patient.

Additionally, in an implementable embodiment, the pulse stimulation device corresponds to two leads 4, an electrode of one lead located in the interventricular septum of the right ventricle (the interventricular septum of the right ventricle as an endocardial location) or in/near the anterior or posterior interventricular sulcus (as an epicardial location); an electrode of the other lead located in the anterolateral wall of the left ventricle (as an epicardial or endocardial location).

In an implementable embodiment, the pulse stimulation device corresponds to three leads 4, an electrode of one lead located in the interventricular septum of the right ventricle (the interventricular septum of the right ventricle as an endocardial location) or in/near the anterior or posterior interventricular sulcus (as an epicardial location); an electrode of one lead located in the posterolateral wall of the left ventricle (as an epicardial or endocardial location), and an electrode of the other lead located in the anterolateral wall of the left ventricle (as an epicardial or endocardial location).

It is certain that the number of leads 4 and the preset implantation position can also be replanned according to the conditions, cardiac-related surgeries, needs for circulatory support, and needs for CCM stimulation of different patients.

In an implementable embodiment, the lead 4 of this example comprises a unipolar lead or a bipolar lead, and the cardiac stimulation pulse is transmitted to the corresponding site via the lead 4. (1) When a single unipolar lead is used, CCM is transmitted between a unipolar electrode in contact with the myocardium and an electrode elsewhere in the patient's body. In this case, the unipolar electrode may be an electrode on other electrode leads in the cardiac chamber or blood vessels, a body surface ECG electrode, or a body surface patch electrode for external defibrillation; or an electrode implanted under the skin (e.g., S-ICD) or an electrode implanted in the heart. Alternatively, it can be obtained via other specially designed electrodes. (2) When multiple unipolar leads are used, CCM can be transmitted between two unipolar electrodes in contact with the myocardium, one of which is a cathode and the other an anode. (3) When a bipolar lead is used, only one electrode is in continuous contact with the myocardium (e.g., lead 4 in the interventricular septum of the right ventricle), and CCM can be transmitted between two electrodes or as a unipolar lead (the electrode in contact with the myocardium) in a form that resembles a unipolar setup. (4) For example, in the case of a left ventricular epicardial electrode, CCM can be transmitted between two electrodes or separately as two unipolar leads. (5) It is possible to use both unipolar and bipolar leads, so that there are a variety of possible combinations, which can be determined and adjusted according to actual needs.

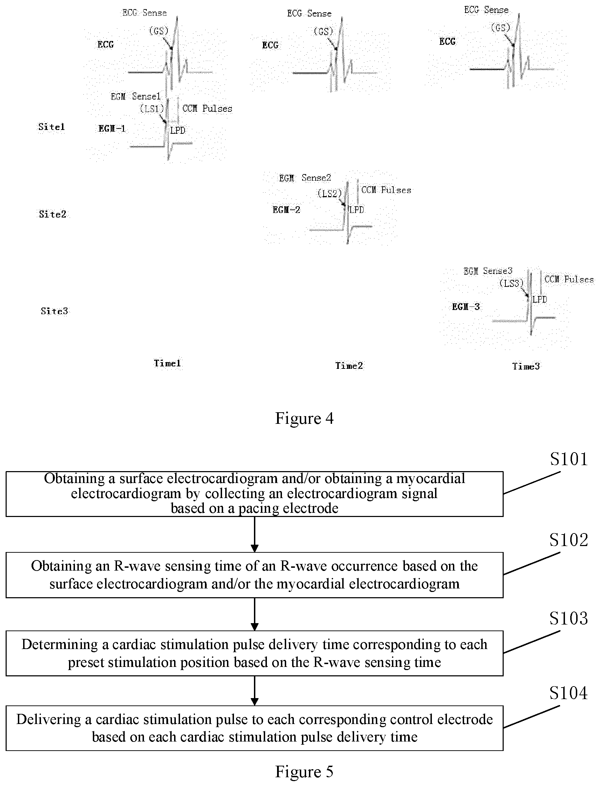

The working principle of the pulse stimulation device of this example is specified below with examples:
(1) Three bipolar leads 4 are implanted on the epicardial surface of a patient who has just undergone open-heart surgery, specifically in the interventricular sulcus of the anterior or posterior extracardiac wall (near the interventricular sulcus area of the ventricle), on the posterolateral wall of the left ventricle, and on the anterolateral wall of the left ventricle; a current surface electrocardiogram of the patient is provided at the same time; the three leads, along with the surface electrocardiogram, are connected to an external electrical circulatory support device, i.e., the R-wave sensing module 1 of the pulse stimulation device;
(2) during the set-up period, the R-wave sensing module 1 is used to sense an R-wave sensing time (GS) of the surface electrocardiogram (ECG) and an R-wave sensing time (LS (LS 1, LS2, LS3)) of the myocardial electrocardiogram (EGM), respectively, as shown in Figure 2; GLSD and GPD (GLSD1 and GPD1, GLSD2 and GPD2, GLSD31 and GPD3) of each site are calculate based on the GS, LS (LS1, LS2, LS3), and LPD, wherein GLSD = LS - GS, GPD = GLSD + LPD;
(3) for a cardiac stimulation pulse delivery pattern (triggered R-wave source, in a synchronized or sequential manner, and in a specific order among the orders or randomized, programmable by all physicians), a cardiac stimulation pulse is generated for one or more than one site based on the cardiac stimulation pulse delivery pattern, LPD, and GPD, as shown in Figure 4;
(4) the device once connected will go through a setup process, comprising sensing the R waves (GS and LS) and calculating the GLSD and GPD for each site (where each of the three leads is located), in the case of GLSDn and GPDn, n = 1, 2, and 3. The setup process is preferably performed during sinus rhythm with no CCM transmission. Then, in case of R-wave triggering pattern of body surface ECG, CCM will be transmitted/triggered in a synchronized manner (at the same heartbeat (R wave)) or a sequential manner (e.g., CCM is transmitted to site 1 after R wave 1, then transmitted to site 2 after R wave 2, and transmitted to site 3 after R wave 3 (one after the other)) or in a random order according to the corresponding GPD at each site after sensing the R wave of the body surface ECG.

Similarly, CCM can be triggered by local R waves at each site in a synchronized or sequential manner (local R-wave pattern).

Additionally, after CCM has been transmitted for a specific number of beats or duration (3600 beats or 60 minutes by default, programmable), the setup process will be initiated again to accommodate potential changes in parameters (e.g., GLSD) due to changes in heart rate and/or patient condition (e.g., post-medication). Additionally, CCM transmission can continue based on updated parameters.

With the support of the control mode of the cardiac stimulation pulse triggering as described above, it can satisfy the acute and/or short-term need of a patient and achieve the needed circulatory support provided by single-site electrical stimulation to the heart of a patient, especially for a patient with weak cardiac function (e.g., low cardiac output) who is not suitable for or is not confident in cardiac surgery, it can effectively strengthen the postoperative cardiac function, allowing a faster recovery of the patient, and provide timely and effective support for the patient's cardiac needs; at the same time, with the support of this technology, it also helps patients and doctors to improve confidence in related surgeries; additionally, the existing mode of enhancing cardiac contractility based on drugs often has side effects (such as arrhythmia and increased myocardial oxygen consumption, with a potential increase in mortality rate), but the control mode of the cardiac stimulation pulse triggering of this example is basically free of side effects (heart rate and oxygen consumption remain basically unchanged), and can achieve more timely and effective electrical stimulation and better improve cardiac contractility.

In this example, placing control electrodes (e.g., corresponding to stimulation and/or pacing functions) at multiple sites in the left ventricle and/or right ventricle enables synchronized or sequential cardiac electrical stimulation (CCM) of multiple sites; the triggering mechanism of CCM stimulation is determined by the time of the R-wave sensing of the local cardiac electrical activity and/or the R-wave sensing of the global cardiac electrical activity, and the triggering mode can be dynamically adjusted according to the actual situation to adapt to the changing heart rate and overall cardiac condition of the patient, i.e., it can be adaptively adjusted according to the dynamic change of each patient's own cardiac condition, so as to effectively improve the control effect of CCM stimulation, achieve better cardiac contractility, and better satisfy the acute and/or short-term need of the patient and achieve the needed circulatory support provided by single-site electrical stimulation to the heart of the patient.

### Example 3

A pulse stimulation device of this example is a further improvement of example 2. This example is for considering a CCM stimulation scenario in which one control electrode performs a pacing operation, specifically:
There are two cases for the CCM stimulation scenario during pacing: a) when pacing is delivered by an independent electrode, the control principle of cardiac stimulation pulse triggering is consistent with the above description and will not be affected; b) when pacing is delivered by a CCM stimulation electrode, the electrode that mainly provides CCM stimulation also has to provide pacing (identified as an electrode position corresponding to LS1 during pacing), and the working principle of the other remaining electrode positions (only providing CCM stimulation) is consistent with the above description. For the CCM stimulation electrode that provides pacing, LS1 during pacing is a pacing pulse delivery time, and the preset duration needs to be extended to 40 to 100 ms, preferably 60 to 80 ms (also programmable and adjustable). In both cases, the set-up period requires two measurements, one at the time of detection of self heart rate (sinus rate) and the other at the time of pacing.

Specifically, when one control electrode performs a pacing operation, the R-wave sensing module is also used to obtain a myocardial electrocardiogram corresponding to all other remaining preset stimulation positions when pacing is performed at one preset stimulation position, and to obtain a new first R-wave sensing time of the R-wave occurrence in the myocardial electrocardiogram and a new second R-wave sensing time of the R-wave occurrence in the surface electrocardiogram;
the R-wave sensing module is also used to calculate a new first pulse delivery time corresponding to each preset stimulation position based on the new first R-wave sensing time and the preset duration;
the R-wave sensing module is also used to calculate a new first time difference between the new second R-wave sensing time and the new first R-wave sensing time of each preset stimulation position, and to calculate a new second pulse delivery time corresponding to each preset stimulation position based on the new first time difference and the preset duration, with the new second R-wave sensing time as a reference zero point.

In this example, when pacing is performed via one control electrode, it is necessary to recalculate a new R-wave sensing time of the R wave in the myocardial electrocardiogram as well as the R wave in the surface electrocardiogram in this pulse stimulation scenario, and then to calculate the corresponding first pulse delivery time and the second pulse delivery time, respectively, so as to realize timely delivery of pulse stimulation in this scenario.

### Example 4

A pulse stimulation device of this example is a further improvement of example 2, specifically:
The R-wave sensing module is used to obtain multiple first R-wave sensing times corresponding to an R-wave occurrence in a myocardial electrocardiogram at multiple preset stimulation positions, to select one first R-wave sensing time as a reference zero point, to calculate a second difference between each first R-wave sensing time following the reference zero point and the reference zero point, and to calculate a first pulse delivery time corresponding to each preset stimulation position based on the second difference and a preset duration.

The R-wave sensing module is used to randomly select one first R-wave sensing time from multiple first R-wave sensing times with times of occurrence, or to select the first R-wave sensing time with the earliest time of occurrence as the reference zero point. Based on practical experience, the first R-wave sensing time with the earliest time of occurrence is generally preferred as the reference zero point.

In this example, the pulse delivery time of each pulse stimulation position can be obtained by selecting the first R-wave sensing time (LS) with the earliest time of occurrence as a reference zero point, using the reference zero point as the basis for the delivery time of other later stimulation positions, calculating the difference between the delivery time and the reference zero point, and adding the preset duration, so as to achieve the timely and effective pulse delivery based only on the myocardial electrocardiogram without relying on the surface electrocardiogram, making the process of controlling the pulse stimulation more flexible and adaptable to more usage scenarios.

### Example 5

A pulse stimulation method of this example is implemented using the pulse stimulation device of example 1.

As shown in Figure 5, the pulse stimulation method of this example comprises:
S101: obtaining a surface electrocardiogram and/or obtaining a myocardial electrocardiogram by collecting an electrocardiogram signal based on a control electrode;
S102: obtaining an R-wave sensing time of an R-wave occurrence based on the surface electrocardiogram and/or the myocardial electrocardiogram;
S103: determining a pulse delivery time corresponding to each preset stimulation position based on the R-wave sensing time; and
S104: delivering a cardiac stimulation pulse to each corresponding control electrode based on each pulse delivery time.

The implementation principle of the pulse stimulation method of this example corresponds to the working principle of the pulse stimulation device in any one of examples 1 to 4, and therefore will not be repeated herein.

With the support of the control mode of the cardiac stimulation pulse triggering as described above, it can satisfy the acute and/or short-term need of a patient and achieve the needed circulatory support provided by single-site electrical stimulation to the heart of a patient, especially for a patient with weak cardiac function (e.g., low cardiac output) who is not suitable for or is not confident in cardiac surgery, it can effectively strengthen the postoperative cardiac function, allowing a faster recovery of the patient, and provide timely and effective support for the patient's cardiac needs; at the same time, with the support of this technology, it also helps patients and doctors to improve confidence in related surgeries; additionally, the existing mode of enhancing cardiac contractility based on drugs often has side effects (such as arrhythmia, accelerated heart rate, and increased myocardial oxygen consumption, with a potential increase in mortality rate), but the control mode of the cardiac stimulation pulse triggering of this example is basically free of side effects (heart rate and oxygen consumption remain basically unchanged), and can achieve more timely and effective electrical stimulation and better improve cardiac contractility.

### Example 6

A medical apparatus of this example comprises the pulse stimulation device in any one of examples 1 to 4.

The medical apparatus may comprise only the pulse stimulation device for use in configuration of various leads; it can also be integrated into or as an accessory to other systems. Such medical apparatus may include, but are not limited to:
(1) An temporary pacing system: ECS function can be added to the temporary pacing system, which is commonly used to provide bradycardia pacing in the patient population as described above. ECS can include a control electrode for pacing as part of a CCM stimulation electrode, or the control electrode can be independent of the CCM stimulation electrode, which minimizes the impact on clinical practice but can provide additional clinical benefit or use additional leads in a position where better enhancement of contraction due to CCM is needed.
(2) A partially or fully implantable device that provides acute/short-term or chronic (long-term) mechanical circulatory support: appropriate leads and/or myocardial electrodes (in the desired position) can be added to such a system to provide ECS and other functions such as bradycardia pacing, ATP, and defibrillation.
(3) An external defibrillator system: such as a wearable defibrillator, an AED, or a defibrillator for use in the emergency room and/or ambulance. CCM can be delivered via a skin electrode (e.g., a defibrillation electrode) after sensing an R wave of a body surface ECG. Only ECS circuits may need to be added to the existing apparatus design or a separate ECS unit can be connected to the current apparatus for this purpose. When a patient is in severe bradycardia or cardiac arrest following an electric shock or in electromechanical dissociation (EMD), the ECS function may provide a more effective aid in the recovery of the patient's cardiac function.
(4) An S-ICD system: R-wave sensing can be achieved by an ECG of a non-myocardial contact electrode (e.g., right ventricular sub-Q electrode pair) or a right ventricular EGM of S-ICD with right ventricular leadless pacemaker, thus CCM stimulation in the S-ICD is triggered and transmitted via a subQ defib electrode and/or a leadless pacemaker electrode. When a patient is in severe bradycardia or cardiac arrest following an electric shock or in electromechanical dissociation, the function may provide a more effective aid in the recovery of the patient's cardiac function.

The medical apparatus of this example comprises the pulse stimulation device as described above and enables cardiac electrical stimulation (CCM) of multiple sites. Compared with the existing mode of merely stimulating the interventricular septum of the right ventricle, it can more effectively improve the overall cardiac contractility (especially the left ventricular contractility) of the patient, and thus can be well suited for a patient who suffers from an acute and/or short-term significant decline in ventricular function, greatly improving the overall product performance of the medical apparatus.

Although the specific embodiments of the present disclosure have been described above, it should be understood by those skilled in the art that these are merely illustrative examples and that a variety of changes or modifications can be made to these embodiments without departing from the principles and essence of the present disclosure. Therefore, the scope of protection of the present disclosure is limited by the appended claims.

## Claims

1. A pulse stimulation device, comprising an R-wave sensing module, a cardiac stimulation pulse generator, and at least one control electrode, wherein each control electrode corresponds to a different preset stimulation position;
the control electrode is electrically connected to the R-wave sensing module and the cardiac stimulation pulse generator, respectively, and the R-wave sensing module is communicatively connected to the cardiac stimulation pulse generator;
the R-wave sensing module is used to obtain a surface electrocardiogram and/or obtain a myocardial electrocardiogram by collecting an electrocardiogram signal based on the control electrode, obtain an R-wave sensing time of a corresponding R-wave occurrence based on the surface electrocardiogram and/or the myocardial electrocardiogram, and determine a pulse delivery time corresponding to each preset stimulation position according to the R-wave sensing time;
the cardiac stimulation pulse generator is used to deliver a cardiac stimulation pulse to each corresponding control electrode according to each pulse delivery time.

2. The pulse stimulation device according to claim 1, **characterized in that** the device comprises at least two control electrodes, and different control electrodes are used for being implanted at different preset stimulation positions in the left ventricular myocardium and/or right ventricular myocardium of a patient.

3. The pulse stimulation device according to claim 1 or 2, **characterized in that** the device further comprises a pulse control module, and the pulse control module is communicatively connected to the cardiac stimulation pulse generator;
the pulse control module is used to generate a pulse delivery pattern and send the pulse delivery pattern to the cardiac stimulation pulse generator;
the cardiac stimulation pulse generator is used to deliver a cardiac stimulation pulse to each corresponding control electrode based on the pulse delivery pattern and each pulse delivery time upon the occurrence of an R wave in the surface electrocardiogram and/or the myocardial electrocardiogram.

4. The pulse stimulation device according to claim 3, **characterized in that** the pulse delivery pattern comprises delivering a cardiac stimulation pulse to the control electrode corresponding to each preset stimulation position in a synchronized manner and in a set or random order, based on the R wave in the surface electrocardiogram and/or the myocardial electrocardiogram.

5. The pulse stimulation device according to claim 3, **characterized in that** the pulse control module is also used to generate a stimulation combination corresponding to different preset stimulation positions based on a set number of the preset stimulation positions, using a set construction rule or a random combination;
wherein the stimulation combination comprises at least two stimulation units, and at least one of the stimulation units corresponds to two or more of the preset stimulation positions where the pulse is delivered in a synchronized manner;
the pulse delivery pattern comprises delivering a cardiac stimulation pulse to the control electrode corresponding to the preset stimulation position in accordance with a set or random order and the stimulation combination, based on the R wave in the surface electrocardiogram and/or the myocardial electrocardiogram.

6. The pulse stimulation device according to claim 4 or 5, **characterized in that** the cardiac stimulation pulse is successively delivered to the corresponding preset stimulation position in a set or random order to achieve a preset number of heartbeats.

7. The pulse stimulation device according to at least one of claims 1 to 6, **characterized in that** the preset stimulation position comprises at least one of an inner wall of the interventricular septum of the left ventricle and/or right ventricle, an interventricular sulcus of the outer wall of the ventricle, an lateral wall of the left ventricle, an anterolateral wall of the left ventricle, a posterolateral wall of the left ventricle, an inner wall of a right ventricular free wall, an outer wall of the right ventricular free wall, a right ventricular apex, and a left ventricular apex.

8. The pulse stimulation device according to at least one of claims 1 to 7, **characterized in that** the R-wave sensing module is used to obtain a surface electrocardiogram and a myocardial electrocardiogram based on the control electrode, and to obtain a first R-wave sensing time of the R-wave occurrence based on the myocardial electrocardiogram and a second R-wave sensing time of the R-wave occurrence based on the surface electrocardiogram;
the R-wave sensing module is also used to calculate a first pulse delivery time corresponding to each preset stimulation position based on the first R-wave sensing time;
the R-wave sensing module is also used to calculate a second pulse delivery time corresponding to each preset stimulation position based on the first R-wave sensing time and the second R-wave sensing;
the cardiac stimulation pulse generator is used to deliver a cardiac stimulation pulse to each corresponding control electrode based on the first pulse delivery time and/or the second pulse delivery time.

9. The pulse stimulation device according to claim 8, **characterized in that** the R-wave sensing module is also used to calculate the first pulse delivery time corresponding to each preset stimulation position based on a preset duration, with the first R-wave sensing time as a reference zero point;
the R-wave sensing module is also used to calculate a first time difference between the second R-wave sensing time and the first R-wave sensing time of each preset stimulation position, and to calculate the second pulse delivery time corresponding to each preset stimulation position based on the first time difference and the preset duration, with the second R-wave sensing time as a reference zero point.

10. The pulse stimulation device according to claim 9, **characterized in that** the cardiac stimulation pulse generator is used to maintain the first time difference after obtaining the second pulse delivery time, so as to maintain delivering a cardiac stimulation pulse to the corresponding control electrode based on the second pulse delivery time.

11. The pulse stimulation device according to claim 9 or 10, **characterized in that** the device further comprises a time update module;
the time update module is used to periodically or irregularly update the first time difference, so as to update the second pulse delivery time based on the updated first time difference;
the cardiac stimulation pulse generator is used to deliver a cardiac stimulation pulse to the corresponding control electrode based on the updated second pulse delivery time.

12. The pulse stimulation device according to at least one of claims 8 to 11, **characterized in that** the R-wave sensing module is also used to obtain the myocardial electrocardiograms corresponding to all other remaining preset stimulation positions when pacing is performed at one preset stimulation position, and to obtain a new first R-wave sensing time of the R-wave occurrence in the myocardial electrocardiogram and a new second R-wave sensing time of the R-wave occurrence in the surface electrocardiogram;
the R-wave sensing module is also used to calculate a new first pulse delivery time corresponding to each preset stimulation position based on the new first R-wave sensing time and the preset duration;
the R-wave sensing module is also used to calculate a new first time difference between the new second R-wave sensing time and the new first R-wave sensing time of each preset stimulation position, and to calculate a new second pulse delivery time corresponding to each preset stimulation position based on the new first time difference and the preset duration, with the new second R-wave sensing time as a reference zero point.

13. The pulse stimulation device according to claim 1, **characterized in that** the R-wave sensing module is used to obtain multiple first R-wave sensing times corresponding to the R-wave occurrences in the myocardial electrocardiograms at multiple preset stimulation positions, to select one first R-wave sensing time as a reference zero point, to calculate a second difference between each first R-wave sensing time following the reference zero point and the reference zero point, and to calculate the first pulse delivery time corresponding to each preset stimulation position based on the second difference and the preset duration.

14. The pulse stimulation device according to claim 13, **characterized in that** the R-wave sensing module is used to randomly select one first R-wave sensing time from multiple first R-wave sensing times with times of occurrence, or to select the first R-wave sensing time with the earliest time of occurrence as the reference zero point.

15. The pulse stimulation device according to any one of claims 8 to 13, **characterized in that** the first R-wave sensing time of the R-wave occurrence obtained in the myocardial electrocardiogram and the second R-wave sensing time of the R-wave occurrence obtained in the surface electrocardiogram correspond to the same heartbeat.

16. The pulse stimulation device according to claim 1, **characterized in that** the control electrode is electrically connected to the R-wave sensing module and the cardiac stimulation pulse generator using a unipolar lead or a bipolar lead.

17. A pulse stimulation method, which is implemented using the pulse stimulation device according to any one of claims 1 to 16, comprising:
obtaining a surface electrocardiogram and/or obtaining a myocardial electrocardiogram by collecting an electrocardiogram signal based on the control electrode;
obtaining an R-wave sensing time of an R-wave occurrence based on the surface electrocardiogram and/or the myocardial electrocardiogram;
determining a pulse delivery time corresponding to each preset stimulation position based on the R-wave sensing time; and
delivering a cardiac stimulation pulse to each corresponding control electrode based on each pulse delivery time.

18. A medical apparatus, comprising the pulse stimulation device according to any one of claims 1 to 16.
